# EUROPEAN PATENT SPECIFICATION

(11) **EP 3 253 395 B1**
(45) Date of publication and mention of the grant of the patent: **29.10.2025**
(21) Application number: 16703837.1
(22) Date of filing: 05.02.2016
(51) Int. Cl.: A61K 35/74, A61K 35/744, A61K 35/747, A61P 1/00

(54) **PROBIOTIC PREPARATION FOR TREATING GUT INFLAMMATION**
PROBIOTISCHES PRÄPARAT ZUR BEHANDLUNG VON DARMENTZÜNDUNGEN
PRÉPARATION PROBIOTIQUE POUR LE TRAITEMENT DE L'INFLAMMATION INTESTINALE

(30) Priority: 05.02.2015 GB 201501938
(43) Date of publication of application: 13.12.2017
(60) Divisional application: 25203836.9
(73) Proprietor: Multigerm UK Enterprises Ltd., Surrey GU10 1PX (GB)
(72) Inventor: SMITH, Barry, Farnham Surrey GU10 1PX (GB); BROADBENT, Robert, Gloucestershire GL54 1DU (GB)
(74) Representative: Boult Wade Tennant LLP
(86) International application number: PCT/GB2016/050269
(87) International publication number: WO 2016/124940

(56) References cited:
- WO-A1-2005/060937
- WO-A1-2006/035218
- WO-A1-2007/036230
- WO-A1-2011/078781
- WO-A1-2013/072654
- GEORGE VAOS ET AL: "The Role of Calprotectin in Pediatric Disease", BIOMED RESEARCH INTERNATIONAL, vol. 2013, 1 January 2013 (2013-01-01), pages 1 - 8, XP055609095, ISSN: 2314-6133, DOI: 10.1155/2013/542363

## Description

### Field of the invention

The invention**,** which is defined in claims 1-14, relates to treatment of inflammatory gut disorders using probiotic preparations containing viable, metabolically active probiotic bacteria.

### Background

Enterocolitis, also described as enterocolitic disorders, encompasses a number of conditions characterised by inflammation of the gastrointestinal (GI) tract. For example, such conditions include those that involve enteritis (inflammation of the small intestine) and/or colitis (inflammation of the colon or large intestine). Enterocolitic inflammation in a patient is indicated by raised inflammatory markers, such as a faecal calprotectin concentration above normal levels (normal level being approximately 50mg/kg).

Enterocolitis may be caused by a number of factors including, but not limited to, infection, autoimmune effects, or as a consequence of pharmacological treatments. For example, infectious enterocolitis may be caused by bacteria such as *Salmonella sp.,* and *Campylobacter sp.,* viruses such as enteroviruses, and parasites such as *Giardia.* Autoimmunity-driven enterocolitic disorders include those such as ulcerative colitis and Crohn's disease (referred to together as inflammatory bowel disease (IBD)). Enterocolitis may also be caused as a consequence or side-effect of pharmacological interventions. For example, raised faecal calprotectin levels are observed in patients chronically treated with non-steroidal anti-inflammatory drugs (NSAIDs).

Patients suffering from enterocolitis may exhibit a range of symptoms, many of which have a significant impact on the patient's quality of life. Such symptoms include, but are not limited to, diarrhoea, vomiting, constipation, abdominal discomfort or pain, rectal bleeding, increased urgency and/or frequency of bowel movements, and anaemia.

In the case of the enterocolitic disorders ulcerative colitis (UC) and Crohn's disease, the incidence in the UK is approximately 10-20 and 5-10 per 100,000 respectively and both diseases are associated with significant morbidity and reduction in quality of life (QOL). Crohn's disease has a particularly significant morbidity, with up to 15% of Crohn's patients not being able to work full-time 5-10 years after diagnosis.

Conventional treatments for entercolitis include the use of various medications to control the infection and/or the inflammatory process and as a result reduce symptoms. However, these are not always effective. For example, in IBD most if not all patients will continue to exhibit raised inflammatory markers and have a reduced quality of life (QOL) secondary to residual symptoms despite treatment. In addition, patients in asymptomatic clinical remission frequently continue to exhibit raised markers of inflammation and as a result are at an increased risk of early relapse.

For example, in IBD, conventional treatments include steroids (like prednisolone and hydrocortisone); the anti-inflammatory 5 amino-salicilates (5-ASAs) (such as mesalazine); immunosuppresants (including, methotrexate, azathioprine, and cyclosporine); and anti-tumour necrosis factors (anti-TNFs,) (such as infliximab and adalimumab).

Whilst current medical therapy has reduced overall morbidity and mortality, 30% of UC patients will ultimately require colectomy and up to 70-80% of Crohn's patients will undergo some form of surgery during their lifetime.

It is clear therefore that alternative or further therapies for treating enterocolitic disorders such as IBD (UC and Crohn's disease) are needed.

WO2006035218 describes a composition comprising viable, metabolically active probiotic bacteria in a non-dairy liquid substrate a growth substrate comprising a mixture of complex and simple carbohydrates.

WO2007036230 describes fermented cereals and probiotic microorganisms as treatment effectors for IBD and IBS.

WO2011078781 describes a non-fermented composition intended to increase the formation of butyric acid in the colon.

WO2005060937 describes compressed tablets comprising viable probiotic microorganisms.

### Summary of the invention

The human intestine supports a diverse population of different bacteria and the symbiotic relationship between the host and this microbiota is essential for normal intestinal health and function. However, disruption of this relationship can lead to the onset of enterocolitic disorders. For example, in IBD the immune system reacts abnormally to the normal intestinal microbiota which ultimately results in the pathological inflammation characteristic of the disease.

One approach to treating such enterocolitic disorders is the use of probiotics. However, the role and efficacy of probiotics in the treatment of enteroclitic disorders such as IBD is still unclear as emerging data from clinical trials to date is limited and conflicting. Poor methodology, lack of randomisation or placebo controls adds further confusion. For example, a recent Cochrane review concluded that 'there is insufficient evidence to make any conclusions about the efficacy of probiotics for induction or remission in Crohn's disease' (Butterworth et al. Cochrane Database of Systematic Reviews 2008, Issue 3. Art No: CD006634). Overall, the variability in study design, response rates, measured clinical parameters and heterogeneity of patient groups between studies has precluded firm conclusions being made about the efficacy of probiotic treatments for enterocolitic disorders such as IBD.

Sisson et al. (Aliment Pharmacol Ther. 2014 Jul;40(1): 51-62) report the use of the multistrain probiotic preparation exemplified herein for the treatment of irritable bowel syndrome (IBS). Such subject-matter is also the subject of WO2013/072654. However, it is noted that IBS is not an enterocolitic disorder.

The effective use of the probiotic preparation exemplified herein in the treatment of irritable bowel syndrome (IBS) gives no indication that the preparation would be of use in the treatment of enterocolitis. IBS is a functional disorder and is characterised by, amongst other factors, an absence of inflammatory markers (for example, IBS patients exhibit normal faecal calprotectin levels (i.e. less than about 50mg/kg). Therefore, whilst the preparation described in Sisson *et al.* may be effective at treating IBS, the skilled person is given no motivation to try such a probiotic preparation in the treatment of enterocolitic disorders, and would have no expectation that it would be efficacious.

As described herein, a randomised, double-blind, placebo controlled trial was carried out to assess the efficacy of a non-dairy, liquid-based probiotic preparation characterised by the presence of a high count of viable, metabolically active probiotic bacteria in treating enterocolitis, specifically UC and Crohn's disease. The data presented herein demonstrate that following treatment with the probiotic preparation, patients exhibited substantially reduced inflammatory markers, indicative of an effective treatment of the underlying enterocolitic disorder.

Therefore, in a first aspect the invention provides a probiotic preparation comprising a mixture of viable, metabolically active *Enterococcus faecium, Lactobacillus plantarum, Lactobacillus acidophilus* and either *Lactobacillus casei* or *Lactobacillus rhamnosus* or both *Lactobacillus casei* and *Lactobacillus rhamnosus* in a non-dairy liquid substrate, for use in the treatment of enterocolitis in a patient having a faecal calprotectin level of greater than 50mg/kg.

In a second aspect, the invention provides a probiotic preparation for use in the treatment of enterocolitis, the probiotic preparation being obtainable by a method comprising: subjecting malted cereal, optionally barley, to a mashing step to obtain a mixture of complex carbohydrates, simple sugars, proteins and peptides; separating the mixture of complex carbohydrates and simple sugars, proteins and peptides from the spent malted cereal to obtain a growth substrate; inoculating the growth substrate with a mixture of *Enterococcus faecium, Lactobacillus plantarum, Lactobacillus acidophilus* and either *Lactobacillus casei* or *Lactobacillus rhamnosus* or both *Lactobacillus casei* to form a culture and growing said bacteria until they reach a concentration of between 1 x 10⁶ and 1 x 10⁹ colony forming units per millilitre; and cooling the culture to 4°C to obtain the probiotic preparation.

In certain embodiments according to all aspects of the invention, the enterocolitic disorder is inflammatory bowel disease. In certain embodiments, the enterocolitic disorder is ulcerative colitis or Crohn's disease. In certain embodiments, the patient to be treated is in clinical remission, preferably wherein the patient has been in clinical remission for at least 3 months prior to treatment.

In certain embodiments according to all aspects of the invention, the probiotic preparation comprises a mixture of complex carbohydrates and simple sugars, wherein the ratio of total carbohydrate content to reducing sugar content of the probiotic preparation is in the range of from 8:1 to 2:1.

In all aspects of the invention, the probiotic preparation comprises a mixture of *Enterococcus faecium, Lactobacillus plantarum, Lactobacillus acidophilus,* and either *Lactobacillus casei* or *Lactobacillus rhamnosus* or both *Lactobacillus casei* and *Lactobacillus rhamnosus.*

In certain embodiments according to all aspects of the invention, the total count of at least one of, and more preferably each of *Enterococcus faecium, Lactobacillus plantarum,* and either *Lactobacillus casei* or *Lactobacillus rhamnosus* or a combination of *Lactobacillus casei* and *Lactobacillus rhamnosus* in the preparation is at least 1.0 x 10⁶ viable cells per millilitre, preferably at least 1.0 x 10⁷ viable cells per millilitre. In certain embodiments, the total count of metabolically active bacteria in the preparation is in the range of 1.0 x 10⁶ to 1.0 x 10⁹ viable cells per millilitre.

In certain embodiments according to all aspects of the invention, the probiotic preparation has a total carbohydrate content in the range of 20 mg/ml to 40 mg/ml and a reducing sugar content in the range of 5 mg/ml to 20 mg/ml. In certain embodiments, the probiotic preparation comprises proteins and peptides, wherein the total amount of protein and peptides is in the range of from 1 mg/ml to 2 mg/ml and wherein the total amount of high molecular weight peptides is in the range of 100 µg/ml to 300 µg/ml. In certain embodiments, the pH of the preparation is maintained in the range of from 3.8 to 4.5. In certain embodiments, the probiotic preparation comprises an anti-oxidant, for example vitamin C. In certain embodiments, the probiotic preparation comprises an anti-fungal agent, for example sterilised potassium sorbate.

### Detailed description of the invention

The invention provides effective treatment of enterocolitic disorders using a probiotic preparation which is demonstrated as providing effective reduction of gut inflammation.

The probiotic preparation used in the effective treatment of enterocolitis is a liquid-based product (and typically a water-based product) which is non-dairy and is not freeze-dried. The probiotic preparation contains viable, metabolically active probiotic bacteria in a liquid substrate which is capable of delivering viable, metabolically active probiotic bacteria to the intestinal tract, where the probiotic bacteria rapidly begin to establish and multiply. The probiotic bacteria in the preparation are therefore "alive" and ready to function immediately after the preparation is swallowed.

Prior art probiotic products are predominately available in dairy or freeze-dried formats such as powders or capsules. In order for probiotics to work effectively and to their optimum, they need to be directed to specific sites within the intestinal tract without triggering digestion as well as avoiding extremes. If digestion is triggered, the stomach acids can weaken or destroy probiotic bacteria. For some probiotics this is a challenge as they are contained in yoghurt type drinks which the stomach initially 'sees' as food. On the other hand, freeze-dried probiotics have been taken down to a temperature of around minus 80°C and the fimbria can be broken off during the freezing process. Freeze-dried probiotics have to rehydrate before they are able to function adequately. This can take several hours. Finding an effective "delivery system" for probiotic bacteria is a key objective for manufacturers producing probiotic supplements or functional food.

The probiotic preparation described herein and shown to be clinically effective in the treatment of enterocolitis does not suffer from these drawbacks, as the probiotic bacteria are carried in a liquid-based substrate, typically a water-based substrate, which shields and transports the probiotic bacteria safely through the stomach (without triggering digestion) to the intestines where they rapidly begin to establish and multiply. Without wishing to be bound by any particular theory, it is believed that the viable nature of the probiotic bacteria in the preparation described herein and shown to be clinically effective in the treatment of enterocolitis allows them to establish rapidly in the patient's gastrointestinal tract, perhaps within 15 to 20 minutes of ingestion. It is believed that such rapid establishment contributes to the efficacy observed in the clinical treatment of entercolitis, resulting in a significant reduction in gut inflammatory markers.

### Definitions

"Probiotic" - as used herein the term "probiotic" is to be interpreted according to the FAO/WHO joint report and guidelines for use of probiotics, in which probiotics are defined as "live microorganisms which when administered in adequate amounts confer a health benefit to the host". The term "probiotic bacteria" refers to any bacterial strain which fulfils this definition of a "probiotic".

"Lactic acid bacteria (LAB)" - as used herein the term "lactic acid bacteria (LAB)" refers to a group of Gram positive, catalase negative, non-motile anaerobic bacteria that ferment carbohydrates to lactic acid. This group includes the genera *Lactobacillus, Lactococcus, Pediococcus, Bifidobacterium, and Enterococcus.*

"Probiotic lactic acid bacteria" - as used herein the term "probiotic lactic acid bacteria" refers to lactic acid bacteria which also satisfy the definition of a "probiotic" as used herein. Exemplary probiotic lactic acid bacteria include, but are not limited to, those in the genera *Lactobacillus* and *Enterococcus.*

"Enterocolitis" and "enterocolitic disorders" - as used herein, "entercolitic disorders" and "enterocolitis" are used interchangeably to refer to inflammatory disorders of the gut - i.e. those disorders in which the small intestine, large intestine, or both, are inflamed. These disorders include colitis disorders (e.g. ulcerative colitis (UC), ischaemic colitis, infectious colitis), enteritis, and conditions in which multiple parts of the gastrointestinal tract are inflamed (e.g. Crohn's disease (CD)). Enterocolitis (or enterocolitic disorders) as used herein includes inflammatory bowel disease (IBD). Enterocolitis (or enterocolitic disorders) as used herein does not encompass the functional disorder irritable bowel syndrome (IBS). An enterocolitic disorder may be caused by infection, autoimmunity, and/or pharmacological action (e.g. through use of non-steroidal anti-inflammatory drugs (NSAIDs)).

Enterocolitis (or an "enterocolitic disorder") as used herein is characterised by a faecal calprotectin level of greater than 50mg/kg. Patients having an enterocolitic disorder may have a faecal calprotectin level greater than 100mg/kg, greater than 150mg/kg, preferably greater than 250mg/kg.

"Inflammatory bowel disease (IBD)" - IBD, as used herein, encompasses ulcerative colitis and Crohn's disease. Crohn's disease and ulcerative colitis are each chronic relapsing and remitting inflammatory diseases characterised by the symptoms including but not limited to abdominal pain or discomfort, diarrhoea, weight loss, rectal bleeding and fatigue. Crohn's disease can affect any part of the gastrointestinal (GI) tract, and is characterised by patchy, trans-mural inflammation. In UC, the inflammation is more superficial but continuous, extending proximally from the rectum and generally limited to the colon. IBD (including each of UC and Crohn's disease) is characterised by a faecal calprotectin level of greater than 50mg/kg, greater than 100mg/kg, greater than 150mg/kg, preferably greater than 250mg/kg.

Patients with IBD (Crohn's disease or UC) can be in a status of clinical remission, or be undergoing a relapse/episode. A patient in clinical remission is characterised by no progressive deterioration in condition (histological or in symptom severity) for a period of at least 1 month. A patient in relapse (i.e. undergoing an episode) is characterised by deterioration in symptoms experienced (i.e. becoming more severe and/or more frequent) and deterioration in histological condition.

"Faecal calprotectin level" - as used herein, faecal calprotectin level refers to the concentration of calprotectin measured in a sample of faeces from a subject. The calprotectin concentration can be determined by methods known in the art and familiar to the skilled person, such as by enzyme-linked immunosorbant assay (ELISA). Calprotectin measurement can be performed using commercially available kits such as the EK-Cal kit from Buhlmann (Switzerland) or the Calprest kit from Eurospital (Italy).

"Treatment of enterocolitis" - as used herein, "treatment of enterocolitis" is characterised by a reduction in the faecal calprotectin level exhibited by the patient. A reduction in faecal calprotectin level is considered to be a fall in faecal calprotectin concentration greater than the background variation in the assay used to determine said concentration. The skilled person is aware how to determine the background variation for any given assay. Treatment of enterocolitis may also be characterised by a reduction in faecal calprotectin level to below a threshold value, where the patient had a faecal calprotectin level equal to or above this threshold value prior to treatment.

Treatment of enterocolitis may be further characterised by a reduction in one or more symptoms exhibited by the patient suffering from the enterocolitic disorder. Such a reduction in a symptom may be characterised by the symptom being less frequent, or less severe, or both.

"Treatment of inflammatory bowel disease" - as used herein, "treatment of inflammatory bowel disease" is characterised by a reduction in the faecal calprotectin level exhibited by the patient post-treatment compared to the faecal calprotectin level exhibited before treatment. A reduction in faecal calprotectin level is considered to be a reduction in faecal calprotectin concentration greater than the background variation in the assay used to determine said concentration. Treatment of IBD may also be characterised by a reduction in faecal calprotectin level to below a threshold value, where the patient had a faecal calprotectin level equal to or above this threshold value prior to treatment.

Treatment of IBD may be further characterised by a reduction in one or more symptoms exhibited by the patient. Such a reduction in a symptom may be characterised by the symptom being less frequent, or less severe, or both. For example, treatment of IBD may be evidenced by less frequent abdominal pain episodes, wherein those episodes are also less painful (i.e. less severe).

Treatment of IBD may also by characterised by longer time periods between relapses. That is, treatment results in a longer average time period between relapses compared to the average time period for that patient before treatment.

"Treatment of ulcerative colitis" - as used herein, "treatment of ulcerative colitis" is characterised by a reduction in the faecal calprotectin level exhibited by the patient post-treatment compared to the faecal calprotectin level exhibited before treatment. A reduction in faecal calprotectin level is considered to be a fall in faecal calprotectin concentration greater than the background variation in the assay used to determine said concentration. Treatment of UC may also be characterised by a reduction in faecal calprotectin level to below a threshold value, where the patient had a faecal calprotectin level equal to or above this threshold value prior to treatment.

Treatment of UC may be further characterised by a reduction in one or more symptoms exhibited by the patient. Such a reduction in a symptom may be characterised by the symptom being less frequent, or less severe, or both.

Treatment of UC may also by characterised by longer time periods between relapses. That is, treatment results in a longer average time period between relapses compared to the average time period for that patient before treatment.

"Treatment of Crohn's disease" - as used herein, "treatment of Crohn's disease" is characterised by a reduction in the faecal calprotectin level exhibited by the patient post-treatment compared to the faecal calprotectin level exhibited before treatment. A reduction in faecal calprotectin level is considered to be a fall in faecal calprotectin concentration greater than the background variation in the assay used to determine said concentration. Treatment of Crohn's Disease may also be characterised by a reduction in faecal calprotectin level to below a threshold value, where the patient had a faecal calprotectin level equal to or above this threshold value prior to treatment.

Treatment of Crohn's disease may be further characterised by a reduction in one or more symptoms exhibited by the patient. Such a reduction in a symptom may be characterised by the symptom being less frequent, or less severe, or both. For example, treatment of Crohn's disease may be evidenced by less frequent abdominal pain episodes, wherein those episodes are also less painful (i.e. less severe).

Treatment of Crohn's disease may also by characterised by longer time periods between relapses. That is, treatment results in a longer average time period between relapses compared to the average time period for that patient before treatment.

"Treatment of a patient having a faecal calprotectin level of greater than "X"mg/kg" - as used herein, "treatment of a patient having a faecal calprotectin level of greater than "X"mg/kg" is characterised by reducing the faecal calprotectin level in the patient from above Xmg/kg before treatment, to less than or equal to Xmg/kg after treatment. That is, when X=50, treatment of a patient having a faecal calprotectin level of greater than 50mg/kg is characterised by reducing the faecal calprotectin level in the patient from above 50mg/kg before treatment, to less than or equal to 50mg/kg after treatment. When X=250, treatment of a patient having a faecal calprotectin level of greater than 250mg/kg is characterised by reducing the faecal calprotectin level in the patient from above 250mg/kg before treatment, to less than or equal to 250mg/kg after treatment.

"Reducing gut inflammation" - as used herein, a reduction in gut inflammation in a patient with enterocolitis is considered to mean that the faecal calprotectin level exhibited by the patient following treatment is reduced compared to the faecal calprotectin level exhibited immediately prior to treatment.

"Complex carbohydrates" - as used herein the term "complex carbohydrates" includes both oligosaccharides and polysaccharides. "Oligosaccharides" are saccharide polymers containing 3 to 10 saccharide units; whereas the term "polysaccharides" includes longer polymeric structures, such as those formed from repeating saccharide (or disaccharide) units.

"Simple sugars" - as used herein the term "simple sugars" refers to both monosaccharides and disaccharides, unless otherwise stated.

"Reducing sugars" - as used herein the term "reducing sugars" refers to sugars which either have an aldehyde group or are capable of forming an aldehyde group in solution through isomerisation. The presence of reducing sugars may be determined by means of the Nelson-Somogyi method using glucose as the reference standard (Somogyi, M. (1052) Journal of Biological Chemistry., Vol. 195., p.19; reproduced in many standard textbooks of carbohydrate chemistry). Although certain complex carbohydrates (e.g. starches) may contain reducing ends, and therefore fulfil the definition of "reducing sugars", a determination of the content of "reducing sugars" in a given sample (e.g. a sample of probiotic preparation as described herein) using the Nelson-Somogyi method may be taken as an approximation of the amount of simple sugars in the sample, since the simple sugars contain a greater proportion of reducing ends per unit mass than complex carbohydrates.

"Total carbohydrate content" - as used herein the terms "total carbohydrate" or "total carbohydrate content" refer to the total amount of complex carbohydrate and simple sugars present in a given product (e.g. a probiotic preparation as described herein). Total carbohydrate content may be measured using the phenol-sulphuric acid assay, using glucose as a reference standard (Dubois, M., Gilles, K. A., Hamilton, J. K., Rebers, P. A. and Smith, F. (1956) Analytical Chemistry, vol. 28., p. 350).

Where reference is made herein to the ratio of total carbohydrate content to reducing sugar content of a liquid-based product (e.g. in a probiotic preparation) then this is to be determined by calculating the ratio of total carbohydrate content of the product, as measured by the phenol-sulphuric acid method described herein (result expressed in mg/ml), to reducing sugar content of the product, as measured by the Nelson-Somogyi method described herein (result expressed in mg/ml).

"Non-dairy" - as used herein the term "non-dairy" refers to products which do not contain and are not based upon milk from a mammal, in accordance with the definition accepted in the art. Accordingly, non-dairy products do not contain and are not based upon milk, butter, cheese (including vegetarian cheese), yoghurt, cream, milk powder, whey, lactose, lactoproteins (including caseins and caseinates), anhydrous milk fat or kephir.

### Treatment of enterocolitis

The probiotic preparations for use according to the claims provide treatment of enterocolitis in a patient having a faecal calprotectin level of greater than 50mg/kg. In certain embodiments, the enterocolitic disoder is selected from ischaemic colitis, infectious colitis, enteritis, and IBD (i.e. Crohn's disease or ulcerative colitis (UC)). In certain embodiments, the enterocolitis is caused by one or more of infection, autoimmunity, or pharmacological action (e.g. through use of non-steroidal anti-inflammatory drugs (NSAIDs)).

The treatment of the enterocolitic disorder is characterised by a reduction in the faecal calprotectin level exhibited by the patient. In such embodiments, a reduction in faecal calprotectin concentration is a reduction greater than the background variation in the assay used to determine said concentration. In certain embodiments, successful treatment is characterised by a reduction in faecal calprotectin of at least 10mg/kg, optionally at least 20 mg/kg, optionally at least 30mg/kg, optionally at least 50mg/kg, optionally at least 100mg/kg, optionally at least 150mg/kg, optionally at least 200mg/kg, optionally at least 250mg/kg, optionally at least 300 mg/kg compared to the faecal calprotectin level prior to treatment.

In certain embodiments, successful treatment is characterised by a reduction in faecal calprotectin level of at least 1%, optionally at least 2%, optionally at least 5%, optionally at least 10%, optionally at least 15%, optionally at least 20% compared to the faecal calprotectin level exhibited by the patient immediately prior to treatment. In certain preferred embodiments, successful treatment is characterised by a reduction in faecal calprotectin concentration of at least 20%.

In certain alternative embodiments, treatment of enterocolitis is characterised by a reduction in faecal calprotectin level to below a threshold value, where the patient had a faecal calprotectin level equal to or above this threshold value prior to treatment. The threshold value may be 50mg/kg, 100mg/kg, 150mg/kg, 200mg/kg, or 250mg/kg faecal calprotectin. In certain preferred embodiments, the faecal calprotectin level following treatment is less than 50mg/kg - i.e. is reduced to a normal (i.e. healthy) level.

In certain preferred embodiments, the enterocolitic disorder is IBD. In certain preferred such embodiments, the enterocolitic disorder is Crohn's disease. In certain alternative preferred embodiments, the enterocolitic disorder is ulcerative colitis (UC). In certain such embodiments, treatment of IBD is characterised by a reduction in the faecal calprotectin level exhibited by the patient. In such embodiments, a reduction in faecal calprotectin level is a fall in faecal calprotectin concentration greater than the background variation in the assay used to determine said concentration. In certain preferred embodiments, treatment of IBD is characterised by a reduction in faecal calprotectin of at least 10mg/kg, optionally at least 20 mg/kg, optionally at least 30mg/kg, optionally at least 50mg/kg, optionally at least 100mg/kg, optionally at least 150mg/kg, optionally at least 200mg/kg, optionally at least 250mg/kg, optionally at least 300mg/kg compared to the faecal calprotectin level prior to treatment.

In certain embodiments, successful treatment is characterised by a reduction in faecal calprotectin level of at least 1%, optionally at least 2%, optionally at least 5%, optionally at least 10%, optionally at least 15%, optionally at least 20% compared to the faecal calprotectin level exhibited by the patient immediately prior to treatment. In certain preferred embodiments, successful treatment is characterised by a reduction in faecal calprotectin concentration of at least 20%.

In certain alternative embodiments, treatment of IBD is characterised by a reduction in faecal calprotectin level to below a threshold value, where the patient had a faecal calprotectin level equal to or above this threshold value prior to treatment. The threshold value may be 50mg/kg, 100mg/kg, 150mg/kg, 200mg/kg, or 250mg/kg faecal calprotectin. In certain preferred embodiments, the faecal calprotectin level following treatment is less than 50mg/kg - i.e. is reduced to a normal (i.e. healthy) level.

In certain embodiments, treatment of IBD may be further characterised by a reduction in one or more symptoms exhibited by the patient. In such embodiments, the symptom or symptoms exhibited by the patient may be selected from abdominal pain, abdominal discomfort, diarrhoea, vomiting, constipation, rectal bleeding, abnormal frequency of bowel movements, abnormal urgency of bowel movements, and anaemia. In certain such embodiments, a reduction in a symptom may be characterised by the symptom being less frequent, or less severe, or both. For example, treatment of IBD may be evidenced by less frequent abdominal pain episodes, wherein those episodes are also less painful (i.e. less severe).

In certain embodiments, treatment of IBD may also be characterised by longer time periods between relapses. That is, treatment results in a longer average time period between relapses compared to the average time period for that patient before treatment.

It will be appreciated that such manifestations of treatment may occur alone or in any combination. For example, a successfully treated patient may exhibit a fall in faecal calprotectin level of 50mg/kg as well as less frequent and less severe abdominal pain.

In certain preferred embodiments, the enterocolitic disorder is ulcerative colitis (UC). In certain such embodiments, treatment of UC is characterised by a reduction in the faecal calprotectin level exhibited by the patient. In such embodiments, a reduction in faecal calprotectin level is a fall in faecal calprotectin concentration greater than the background variation in the assay used to determine said concentration. In certain preferred embodiments, treatment of UC is characterised by a reduction in faecal calprotectin of at least 10mg/kg, optionally at least 20 mg/kg, optionally at least 30mg/kg, optionally at least 50mg/kg, optionally at least 100mg/kg, optionally at least 150mg/kg, optionally at least 200mg/kg, optionally at least 250mg/kg, optionally at least 300mg/kg compared to the faecal calprotectin level prior to treatment.

In certain embodiments, successful treatment is characterised by a reduction in faecal calprotectin level of at least 1%, optionally at least 2%, optionally at least 5%, optionally at least 10%, optionally at least 15%, optionally at least 20% compared to the faecal calprotectin level exhibited by the patient immediately prior to treatment. In certain preferred embodiments, successful treatment is characterised by a reduction in faecal calprotectin concentration of at least 20%.

In certain alternative embodiments, treatment of enterocolitis is characterised by a reduction in faecal calprotectin level to below a threshold value, where the patient had a faecal calprotectin level equal to or above this threshold value prior to treatment. The threshold value may be 50mg/kg, 100mg/kg, 150mg/kg, 200mg/kg, or 250mg/kg faecal calprotectin. In certain preferred embodiments, the faecal calprotectin level following treatment is less than 50mg/kg - i.e. is reduced to a normal (i.e. healthy) level.

In certain embodiments, treatment of UC may be further characterised by a reduction in one or more symptoms exhibited by the patient. In such embodiments, the symptom or symptoms exhibited by the patient may be selected from abdominal pain, abdominal discomfort, diarrhoea, vomiting, constipation, rectal bleeding, abnormal frequency of bowel movements, abnormal urgency of bowel movements, and anaemia. In certain such embodiments, a reduction in a symptom may be characterised by the symptom being less frequent, or less severe, or both. For example, treatment of UC may be evidenced by less frequent abdominal pain episodes, wherein those episodes are also less painful (i.e. less severe).

In certain embodiments, treatment of UC may also be characterised by longer time periods between relapses. That is, treatment results in a longer average time period between relapses compared to the average time period for that patient before treatment.

It will be appreciated that such manifestations of treatment may occur alone or in any combination. For example, a successfully treated patient may exhibit a fall in faecal calprotectin level of 50mg/kg as well as less frequent and less severe abdominal pain.

In certain preferred such embodiments, the enterocolitic disorder is Crohn's disease. In certain such embodiments, treatment of Crohn's disease is characterised by a reduction in the faecal calprotectin level exhibited by the patient. In such embodiments, a reduction in faecal calprotectin level is a fall in faecal calprotectin concentration greater than the background variation in the assay used to determine said concentration. In certain preferred embodiments, treatment of Crohn's disease is characterised by a reduction in faecal calprotectin of at least 10mg/kg, optionally at least 20 mg/kg, optionally at least 30mg/kg, optionally at least 50mg/kg, optionally at least 100mg/kg, optionally at least 150mg/kg, optionally at least 200mg/kg, optionally at least 250mg/kg, optionally at least 300mg/kg, compared to the faecal calprotectin level prior to treatment.

In certain embodiments, successful treatment is characterised by a reduction in faecal calprotectin level of at least 1%, optionally at least 2%, optionally at least 5%, optionally at least 10%, optionally at least 15%, optionally at least 20% compared to the faecal calprotectin level exhibited by the patient immediately prior to treatment. In certain preferred embodiments, successful treatment is characterised by a reduction in faecal calprotectin concentration of at least 20%.

In certain alternative embodiments, treatment of enterocolitis is characterised by a reduction in faecal calprotectin level to below a threshold value, where the patient had a faecal calprotectin level equal to or above this threshold value prior to treatment. The threshold value may be 50mg/kg, 100mg/kg, 150mg/kg, 200mg/kg, or 250mg/kg faecal calprotectin. In certain preferred embodiments, the faecal calprotectin level following treatment is less than 50mg/kg - i.e. is reduced to a normal (i.e. healthy) level.

In certain embodiments, treatment of Crohn's disease may be further characterised by a reduction in one or more symptoms exhibited by the patient. In such embodiments, the symptom or symptoms exhibited by the patient may be selected from abdominal pain, abdominal discomfort, diarrhoea, vomiting, constipation, rectal bleeding, abnormal frequency of bowel movements, abnormal urgency of bowel movements, and anaemia. In certain such embodiments, a reduction in a symptom may be characterised by the symptom being less frequent, or less severe, or both. For example, treatment of Crohn's disease may be evidenced by less frequent abdominal pain episodes, wherein those episodes are also less painful (i.e. less severe).

In certain embodiments, treatment of Crohn's disease may also be characterised by longer time periods between relapses. That is, treatment results in a longer average time period between relapses compared to the average time period for that patient before treatment.

It will be appreciated that such manifestations of treatment may occur alone or in any combination. For example, a successfully treated patient may exhibit a fall in faecal calprotectin level of 50mg/kg as well as less frequent and less severe abdominal pain.

In certain embodiments, the products and methods of the invention provide treatment for of a patient having a faecal calprotectin level of greater than 50mg/kg. In certain such embodiments, treatment of the patient is characterised by a reduction in faecal calprotectin level to below 50mg/kg. In certain embodiments, the patient has a faecal calprotectin level of greater than 100mg/kg, optionally greater than 150mg/kg, optionally greater than 200mg/kg.

In certain embodiments, the patient has a faecal calprotectin level of greater than 250mg/kg. In such embodiments, treatment of a patient having a faecal calprotectin level of greater than 250mg/kg is characterised by reducing the faecal calprotectin level in the patient from above 250mg/kg before treatment, to less than or equal to 250mg/kg after treatment. In certain embodiments, the patient has a faecal calprotectin level of greater than 700mg/kg. In such embodiments, treatment of a patient having a faecal calprotectin level of greater than 700mg/kg is characterised by reducing the faecal calprotectin level in the patient from above 700mg/kg before treatment, to less than or equal to 700mg/kg after treatment.

In certain embodiments, treatment of the patient may be further characterised by a reduction in one or more symptoms exhibited by the patient. In such embodiments, the symptom or symptoms exhibited by the patient may be selected from abdominal pain, abdominal discomfort, diarrhoea, vomiting, constipation, rectal bleeding, abnormal frequency of bowel movements, abnormal urgency of bowel movements, and anaemia. In certain such embodiments, a reduction in a symptom may be characterised by the symptom being less frequent, or less severe, or both. For example, treatment of Crohn's disease may be evidenced by less frequent abdominal pain episodes, wherein those episodes are also less painful (i.e. less severe).

It will be appreciated that such manifestations of treatment may occur alone or in any combination. For example, a successfully treated patient may exhibit a fall in faecal calprotectin level below the stated threshold as well as less frequent and less severe abdominal pain.

Also provided are probiotic preparations and methods as herein defined for use in reducing gut inflammation in a patient having enterocolitis. In such embodiments, the reduction in gut inflammation is indicated by a reduction in the faecal calprotectin level exhibited by the patient following treatment, when compared to the faecal calprotectin level prior to treatment. In certain such embodiments, the reduction in faecal calprotectin level is greater than the background level of the assay used for the measurements. In certain embodiments, the reduction is characterised by a reduction in faecal calprotectin level of at least 1%, optionally at least 2%, optionally at least 5%, optionally at least 10%, optionally at least 15%, optionally at least 20% compared to the faecal calprotectin level exhibited by the patient immediately prior to treatment. In certain preferred embodiments, successful treatment is characterised by a reduction in faecal calprotectin concentration of at least 20%.

In certain preferred embodiments, the preparation or method is for treating a patient in clinical remission, optionally a patient that has been in clinical remission for at least 3 months. In such cases, clinical remission is defined as set out herein. That is, a patient suffering from enterocolitis (i.e. an enterocolitic disorder such as IBD) is in clinical remission if there has been no progressive deterioration in the enterocolitic disorder (for example histologically and/or in symptom severity) for a period of at least 1 month. Where a patient has been in clinical remission for at least 3 months, the 1 month of non-deterioration required for classification as in clinical remission is included in the 3 month term.

In certain preferred embodiments, the preparation is administered orally. In certain preferred embodiments, the preparation is administered at a dose of 1mg/kg of the patient, at least once a day. In certain embodiments, the preparation is administered for at least 1 month.

In certain embodiments, the probiotic preparation is provided in conjunction with an adjunct therapy, for example a 5-ASA, e.g. mesalazine.

In certain preferred embodiments, the patient is a mammal, preferably a human.

### Probiotic bacterial strains

In each aspect of the invention, the probiotic bacteria included in the probiotic preparation are a combination of *Enterococcus faecium, Lactobacillus plantarum, Lactobacillus acidophilus* and either *Lactobacillus casei* or *Lactobacillus rhamnosus* or both *Lactobacillus casei* and *Lactobacillus rhamnosus.*

Reference to *Lactobacillus casei* is intended to include any bacterial strain originally classified as *Lactobacillus casei* but now re-classified as *Lactobacillus rhamnosus.* Accordingly, reference to *Lactobacillus rhamnosus* may include bacterial strains which have previously been classified as *Lactobacillus casei.*

The product Symprove^{™} (containing *Enterococcus faecium, Lactobacillus plantarum, Lactobacillus casei* and *Lactobacillus acidophilus*) was shown to be particularly effective in reducing gut inflammation in the clinical study described herein. The strain of *Lactobacillus casei* in Symprove^{™} was originally characterised as *Lactobacillus casei* but has now been re-classified as the closely related *Lactobacillus rhamnosus.*

A key factor in achieving therapeutic efficacy in the treatment of enterocolitis, as measured as a reduction in faecal calprotectin levels, is the ability to deliver a high count of viable, metabolically active probiotic bacteria to the gastrointestinal tract of the patient undergoing treatment. In order to achieve this, in exemplary embodiments, the total population of metabolically active bacteria in the probiotic preparation may be in the range of from 1.0 x 10⁶ to 1.0 x 10⁹ viable cells per millilitre, preferably in the range of from 1.0 x 10⁷ to 1.0 x 10⁹ viable cells per millilitre. Each individual strain of metabolically active bacteria present in the probiotic preparation may be present in the range of from 1.0 x 10⁵ to 1.0 x 10⁹ viable cells per millilitre, more preferably in the range of from 1.0 x 10⁷ to 1.0 x 10⁹ viable cells per millilitre.

The probiotic preparations for use according to the claims comprise a combination of *Enterococcus faecium, Lactobacillus plantarum,* and either *Lactobacillus casei* or *Lactobacillus rhamnosus* or both *Lactobacillus casei* and *Lactobacillus rhamnosus,* and it is preferred that at least one and preferably each of these strains is present in the preparation in the range of from 1.0 x 10⁶ to 1.0 x 10⁹ viable cells per millilitre, preferably in the range of from 1.0 x 10⁷ to 1.0 x 10⁹ viable cells per millilitre. The probiotic preparations for use according to the claims also comprise *Lactobacillus acidophilus,* and the population of *L*. *acidophilus* may be lower than 1.0 x 10⁵ viable cells per millilitre.

In an exemplary embodiment the preparation may comprise a combination of *Enterococcus faecium, Lactobacillus plantarum,* and either *Lactobacillus casei* or *Lactobacillus rhamnosus* or both *Lactobacillus casei* and *Lactobacillus rhamnosus,* wherein the bacterial count for each of these bacterial strain is in the range of from 1.0 x 10⁵ to 1.0 x 10⁹ viable cells per millilitre, more preferably in the range of from 1.0 x 10⁷ to 1.0 x 10⁹ viable cells per millilitre.

The probiotic preparations for use according to the claims also contain *Lactobacillus acidophilus.* The population of *L. acidophilus* may be lower than 1.0 x 10⁵ viable cells per millilitre.

Although the total bacterial count in the presently described preparation may appear to be lower than that of typical freeze-dried probiotic preparations, it is important to note that the bacteria are viable. As noted elsewhere herein, it is believed that the viable nature of the bacteria in the probiotic preparation described herein allows them to establish more rapidly in a patient's gastrointestinal tract, perhaps within 15 to 20 minutes of ingestion. It is believed that such rapid establishment contributes to the beneficial effects of the preparation in the treatment of enterocolitis, for example IBD.

Another key advantage of the probiotic preparation described herein, and shown to be useful in the treatment of enterocolitis, is that the probiotic strains are significantly more stable at pH3 than proprietary milk-based or freeze-dried probiotic products, and therefore more likely to be capable of tolerating the harsh conditions encountered in the human GI tract. In particular, it has been shown that the probiotic bacteria in the preparation described herein are stable both at pH6 and at pH3. In exemplary embodiments the probiotic bacteria in the probiotic preparation are stable when maintained in culture at pH 3 for a period of at least 6 hours. In this context "stable" can be taken to mean that when the bacteria are cultured at pH 3, 37ºC, in standard culture medium (e.g. MRS broth) then over a period of at least 6 hours the bacterial count (cfu/ml) does not fall by more than 0.5 log₁₀ units below the bacterial count (cfu/ml) at time zero. In specific embodiments, the bacterial count should remain above 10⁶ cfu/ml for at least 6 hours when cultured at pH 3, 37ºC, in standard MRS broth. In fact, the bacterial count may be observed to increase when cultured under these conditions.

### Composition of the probiotic preparation

The probiotic preparation for use according to the claims and shown herein to provide effective treatment of enterocolitis (for example IBD) comprises a mixture of viable, metabolically active *Enterococcus faecium, Lactobacillus plantarum, Lactobacillus acidophilus* and either *Lactobacillus casei* or *Lactobacillus rhamnosus* or both *Lactobacillus casei* and *Lactobacillus rhamnosus* in a non-dairy liquid substrate. The function of the substrate is to support, and maintain viability of, the probiotic bacteria, particularly during storage of the preparation, such that the probiotic bacteria can be maintained, and then delivered to the patient, in a viable, metabolically active form. To achieve this objective, the liquid substrate typically contains a mixture of complex carbohydrates and simple sugars. In particular, the substrate may comprise a mixture of polysaccharides, oligosaccharides, disaccharides and monosaccharides.

In certain embodiments, the probiotic preparation may be characterised by the ratio of total carbohydrate content to reducing sugar content of the preparation, reflecting the complex mix of polysaccharides, oligosaccharides, disaccharides and monosaccharides present. In exemplary embodiments, the ratio of total carbohydrate content to reducing sugar content of the preparation is in the range of from 8:1 to 2:1, more typically in the range of from 5:1 to 2.5:1, or in the range of from 4:1 to 3:1. Means of measuring the total carbohydrate content and reducing sugar content, and calculating the ratio between them, are as defined above.

In further exemplary embodiments of the probiotic preparation, the total carbohydrate content of the preparation may be in the range of from 20 mg/ml to 40 mg/ml, or in the range of from 20 mg/ml to 30 mg/ml and the total reducing sugar content may be in the range of from 5 mg/ml to 20 mg/ml, or in the range of from 5 mg/mg to 10 mg/ml.

The probiotic preparation preferably also comprises protein and peptide components. Typically the total amount of protein and peptides present in the probiotic preparation is in the range of from 1 mg/ml to 2 mg/ml and the total amount of high molecular weight peptides (molecular weight greater than 5000 Daltons) is in the range of from 100 µg/ml to 300 µg/ml. In a specific embodiment, the concentration of protein and peptides may be about 2mg/ml and the concentration of high molecular weight peptides may be about 250µg/ml.

The probiotic preparation may contain further components such as, for example, cellulose, starch, β-glucans, pentosans, polyphenols, ribonucleic acids, lipids, phosphates, flavenoids, amino acids, vitamins (B₁, B₂, C and E), silicates and trace elements.

The probiotic preparation may comprise an extract of germinated barley containing the desired probiotic bacterial strains.

A probiotic preparation herein described comprises extract of germinated barley and a combination of *Enterococcus faecium, Lactobacillus plantarum,* and either *Lactobacillus casei* or *Lactobacillus rhamnosus* or both *Lactobacillus casei* and *Lactobacillus rhamnosus,* wherein the bacterial count for each of these bacterial strain is in the range of from 1.0 x 10⁵ to 1.0 x 10⁹ viable cells per millilitre, more preferably in the range of from 1.0 x 10⁷ to 1.0 x 10⁹ viable cells per millilitre, and contains *Lactobacillus acidophilus.*

A probiotic preparation herein described for use in the treatment of enterocolitis is a probiotic preparation obtainable by the method described herein below. In certain embodiments, such a preparation comprises a combination of *Enterococcus faecium, Lactobacillus plantarum,* and either *Lactobacillus casei* or *Lactobacillus rhamnosus* or both *Lactobacillus casei* and *Lactobacillus rhamnosus,* wherein the bacterial count for each of these bacterial strain is in the range of from 1.0 x 10⁵ to 1.0 x 10⁹ viable cells per millilitre, more preferably in the range of from 1.0 x 10⁷ to 1.0 x 10⁹ viable cells per millilitre, and contains *Lactobacillus acidophilus.*

Additional components may be added to the probiotic preparation, such as flavourings and/or colourings, to improve palatability for human patients.

The pH of the preparation can be conveniently controlled by the addition of a suitable buffer or combination of buffering agents. Preferred buffers include, for example, tri-sodium citrate or phosphate buffers. The pH of the liquid-based preparation described herein is typically maintained in the range of from 3.8 to 4.5, and in particular at about pH 4.0, during long-term storage. The probiotic preparation may be stored at any temperature from 4°C up to ambient temperature (about 25°C). The Symprove^{™} product described herein has been shown to remain stable (in terms of bacterial count) for a period of at least 6 months when stored at about 4°C, and for at least 4 months when stored at 25°C.

The preparation may additionally comprises an anti-fungal agent, such as, for example, sterilised potassium sorbate and/or and anti-oxidant, such as vitamin C.

In a preferred embodiment the growth substrate may contain particulate matter, for example particles not exceeding 1mm in diameter.

The most preferred embodiment of the probiotic preparation, shown herein to be effective in the treatment of entercolitis as measured by a reduction in faecal calprotectin concentration is the product denoted Symprove^{™}, containing viable, metabolically active cells of *Enterococcus faecium, Lactobacillus plantarum, Lactobacillus casei* and *Lactobacillus acidophilus,* which may be prepared according to the examples provided herein. The strain of *Lactobacillus casei* in Symprove^{™} was originally characterised as *Lactobacillus casei* but has now been re-classified as the closely related *Lactobacillus rhamnosus.*

The Symprove^{™} product is water-based, with a typical water content of about 95% (v/v). This high water content is advantageous since it may result in the product being perceived by the human GI system as a drink rather than "food", thus avoiding triggering digestion.

The probiotic preparation may also be lactose-free, since it is not milk-based, and gluten-free. In particular the Symprove^{™} product is demonstrated to fulfil the accepted criteria for classification as "gluten-free" based on Gliadin ELISA. Batches of the product typically contain less than 10ppm gluten when measured by Gliadin ELISA.

### Manufacture of the probiotic preparation

The probiotic preparation for use in treatment of enterocolitis (for example IBD) as described herein may be prepared by growing one or more probiotic bacterial strains in a liquid growth substrate, such as for example an extract of germinated barley. The growth substrate may be itself prepared starting from seed or malting sample barley using the manufacturing process described herein below. This method is substantially as described in WO 2006/035218.

The growth substrate may be prepared according to a method comprising:
(i) subjecting malted cereal to a mashing step in which the malted cereal is mixed with aqueous liquid and subjected to conditions of time and temperature which limit the extent of conversion of complex carbohydrates to simple sugars such that a mixture of complex carbohydrates, simple sugars, proteins and peptides is obtained; and
(ii) separating the mixture of complex carbohydrates and simple sugars, proteins and peptides from the spent malted cereal to obtain a growth substrate.

The terms "malted cereal" or "malt" as used herein refers to the product of a malting process applied to cereal grains. Malting is a process well known in the art of brewing.

In a typical malting process cereal grains (e.g. seed or malting sample barley) are germinated to induce the mobilisation of storage nutrients. Germinating seeds produce a number of enzymes to mobilise storage proteins and carbohydrates, including □-amylases which hydrolyse starch into maltose. An exemplary cereal is barley, but other cereals, such as rice, wheat, corn and oats or even mixtures thereof, may also be used. The process of germination is generally well known. It will be appreciated that the method may be carried out by providing malted grains or a synthetic substrate comprising a mixture of carbohydrates, proteins and enzymes.

Typically the malting grains are rolled before further processing. Cracking of the grains by rolling facilitates access of water and extraction of nutrients during the mashing step, whilst avoiding shattering of the grains assists in the subsequent separation of the growth substrate from spent malted grains.

The prepared malt is subjected to a mashing step which resembles the mashing-in step used in methods for brewing (see, for example, Kunze, W. Technology Brewing and Malting (1996)). The term "mashing-in" is well known in the field of brewing and relates to a process wherein malted grains are agitated in the presence of water heated to defined temperatures in order to prepare a wort. During the mashing in step complex carbohydrates in the malted grains are broken down into maltose.

The method described herein also involves a mash step in which malted cereal grains are mixed with an aqueous liquid (typically water) and the mixture heated to various defined temperatures. This mash step does not, however, conform to a typical brewer's mashing-in process. Brewers aim to convert as much carbohydrate to simple sugars as possible, for subsequent fermentation to alcohol. In contrast, the conditions of the mash step described herein are specifically chosen to limit the extent of conversion to simple sugars, leaving significant amounts of carbohydrates in more complex oligomeric and polymeric forms.

The extent of conversion of carbohydrate may be limited such that the amount of reducing sugars present in the resulting growth substrate, expressed as a percentage (w/w) of total carbohydrate content, is in the range of from 10% (w/w) to 50% (w/w). As noted above, the reducing sugar content measured by the Nelson-Somogyi method is a good approximation of the total amount of simple sugars present.

The desired limited conversion of complex to simple sugars may be achieved by increasing the temperature in the mash step over a short period of time, typically 30 minutes, without allowing the mixture of malted grains/water to rest at intermediate temperatures. Traditional brewing processes include rests at 60-65°C and 70-74°C, as this allows enzymes to produce high concentrations of simple sugars (mainly maltose). Accordingly, the mashing-in step described herein does not comprise rests at temperatures in the range of from 60°C to 65°C and/or at temperatures in the range of from 70°C to 74°C.

The mash step may comprise mixing the malted cereal with water at a temperature in the range of from 30°C to 45°C, resting the mixture for 1 to 2 hours, increasing the temperature to a temperature in the range of from 75°C to 85°C over a time period in the range of from 20 to 60 minutes, preferably 30 minutes, and then resting the mixture at a temperature in the range of from 75°C to 85°C for a period of time in the range of from 30 to 90 minutes. At the higher temperature, any enzymes present in the mixture are inactivated and nutrients can be extracted. Higher temperatures in the range of from 76°C to 80°C and specifically 78°C are generally preferred. The temperature in this step needs to be sufficiently high for sufficiently long to inactivate all enzymes present in the preparation. It will be appreciated that the precise temperature and times used can vary somewhat according to the type of cereal used.

The process described here specifically limits the amount of conversion of complex sugars to simple sugars. Furthermore, the initial rest at a temperature in the range of from 30°C to 45°C provides an additional advantage in that it maximises the release of amino acids and peptides. Temperatures towards the higher end of this range, i.e. from 40°C to 45°C or specifically about 45°C, are generally preferred. The purpose of this step is to hydrolyse storage proteins present in the cereal grains into available amino acids and peptides. The optimum combination of time and temperature to achieve the desired hydrolysis may vary somewhat depending on the type of grains used.

The presence of high concentrations of proteins, peptides and amino acids is desirable in a growth substrate to be used to support the growth of microorganisms as it provides a useful a source of nitrogen. Typical mashing-in processes used in traditional brewing would generally not include a rest at a temperature in this range, since brewers do not normally seek to achieve high protein or amino acid content in a wort intended for fermentation to produce alcohol.

When the mash step is complete, e.g. all the desired nutrients have been extracted from the now "spent" malted grains, the resulting mixture comprising complex carbohydrates and simple sugars, proteins and peptides may be separated from the spent grains to obtain a growth substrate using any suitable means. Typically this will involve coarse filtration, for example using a 1mm filter such as a wedge-wire basket, yielding a solution containing coarse particles. It is a feature of the process described herein that the growth substrate is *not* clarified, as would generally be the case with a wort prepared during standard brewing. In traditional brewing the wort is generally clarified to remove all coarse particles, thereby producing a clear liquid.

The presence of some particulate matter in a growth substrate prepared according to the process described herein is advantageous as regards subsequent use in supporting the growth of bacteria since it provides both slow release nutrients and particulate surfaces for the adhesion of bacterial cultures.

If required, the growth substrate prepared according to the process described herein can be sterilised prior to further usage. As will be appreciated, this can be carried out by boiling for about one hour or by autoclaving at 120°C for about 20 minutes.

A buffer or several buffering components may be added to the growth substrate if required. Preferred buffers are tri-sodium citrate or phosphate buffers. If the growth substrate is to be sterilised, the buffer(s) may be added prior to, during or after sterilisation as convenient.

The prepared growth substrate is then inoculated with the desired probiotic bacterial strains and grown until they reach the desired bacterial count required in the probiotic preparation. In a specific embodiment, the probiotic bacteria are grown in the growth substrate until they reach a concentration of between 1 x 10⁶ and 1 x 10⁹ colony forming units per millilitre. At this point the culture (or fermentation) can be cooled down to about 4°C and the combination of temperature, pH, phase of growth and residual substrate composition provides equilibrium conditions which allows for maintenance of a near equilibrium growth state during long term storage. In this near equilibrium state, which can be maintained for a period of at least 5-6 months, the population of metabolically active probiotic bacteria is maintained, typically in the range of from 10⁶ to 10⁹ viable cells per millilitre. It will be appreciated that the precise number of viable cells at the near equilibrium state can vary somewhat depending on the species of probiotic bacteria used. Typically the bacterial count (cfu/ml) is "stable" meaning that it does not vary by more than 1 log₁₀ unit, or more preferably by more than 0.5 log₁₀ units, when the product is stored at 4°C for 5 months and/or when stored at 25°C for 4 months.

This manufacturing method involves a growing step in which the probiotic bacteria are grown in a growth substrate until they reach a concentration which enables the near-equilibrium condition to be achieved during subsequent storage. It is an important feature of the method that the growth substrate used provides a balanced nutrient supply containing a mixture of complex carbohydrates and simple sugars wherein a high proportion of carbohydrate content is in the form of complex carbohydrates that may be used as an energy source by the probiotic bacteria. This mix helps to limit immediately available energy during the growing step. The composition and preferred features of the growth substrate is/are preferably as described above. The growth substrate is preferably prepared from malted cereal grains using the manufacturing method described herein, and is typically an extract of germinated barley. It will be appreciated, however, that it is not strictly necessary to use growth substrate prepared according to this method. Similar results can be achieved using growth substrate prepared synthetically by mixing the required proportions of complex carbohydrates and simple sugars, proteins and peptides.

The growing step must be carried out in such a way that care is taken not to grow the probiotic bacteria for too long, to avoid producing acid conditions which would otherwise inhibit further growth as well as limiting the period of storage of the resulting preparation. On the other hand, if the growing step is terminated prematurely, this will limit biomass production. The pH of the preparation during growth of the probiotic bacteria can be used as an indicator of the near equilibrium state. In a typical embodiment, cultures of lactic acid bacteria may be grown until a pH of 4.5 +/- 0.3 units is reached.

It will be appreciated that the pH of the growth substrate may vary somewhat depends on the optimal pH range for the probiotic bacteria used. In a typical embodiment (suitable for lactic acid bacteria) pH should be maintained in the range of from 3.8 to 4.5 during storage. Preferably buffers, such as tri-sodium citrate or phosphates are added to the growth substrate to control the pH during growth of the probiotic bacteria and subsequent storage.

It will be appreciated that the exact timing of the step of growing the probiotic bacteria to a near equilibrium state depends on the species used. Growth of the probiotic bacteria can be carried out in any suitable culture apparatus. In specific embodiments the growing step can be carried out by way of fermentation in a fermentation vessel.

If species of the genera *Enterococcus* and *Lactobacillus* were to be grown using "conventional" growth media consisting largely of fermentable simple sugars the excess energy supply would lead to excess acid production by lactic acid bacteria, which would limit biomass production and shelf life of the resulting culture. In contrast, the mix of complex carbohydrates and simple sugars present in the growth substrate used in the manufacturing method described herein supplies energy for growth in the initial growing step and also for maintenance during storage of the product.

The growing step can be carried out using a single species of probiotic bacteria, and two or more different bacterial species may be grown independently in separate growth media and then blended together before or after the chilling step. The growth substrates used for independent culture of two or more different bacterial species may be the same or of different composition. Thus, it is possible to optimise the growing conditions for cultures of individual bacterial species and then combine the cultures together for storage under conditions which permit maintenance of equilibrium growth for each of the individual species in the culture.

In other manufacturing methods, two or more different bacterial species can be grown together in a single culture in the same growth substrate, provided that their growth rates are comparable so that one species does not dominate the population.

Following the growing step, the probiotic preparation can be analysed by standard methods to determine the microbiological purity and enumeration of probiotic bacteria. It is also possible to grow two or more different combinations of probiotic bacteria together in separate growth substrates and then combine the cultures together in a final probiotic preparation. A combination culture could similarly be blended with a culture of a single bacterial species.

Starter cultures for the growing step of the method include, for example, freeze-dried bacteria or liquid cultures.

It will be appreciated that the probiotic preparation may be used immediately but more typically the preparation will be stored before use. The optimum temperature for storage in order to maintain the near equilibrium growth state in the culture is about 4°C but it will be appreciated by the skilled reader that the storage temperature could vary somewhat. As noted above, the probiotic preparation is stable for at least 4 months even when stored at ambient temperature (about 25°C). For convenience, aliquots of the probiotic preparation produced by the method may be dispensed into suitable sterile packaging prior to long term storage.

It is a key advantage of the probiotic preparation described herein that the product maintains viability and integrity [of the probiotic bacteria] when stored for extended periods, typically for at least 5 or 6 months. However, it will be appreciated that it is not essential to the method that the probiotic preparation must *actually* be stored for 5-6 months prior to use.

To avoid the growth of fungi or yeast, an anti-fungal agent, such as sterile potassium sorbate, may be added prior to storage of the probiotic preparation. The anti-fungal agents are primarily to prevent spoilage by yeast or fungi during use by end-users, once a container of the product has been opened. Furthermore, an anti-oxidant, for example, vitamin C can be added to help to prevent spoilage of the product during storage. It will be appreciated that other agents well known in the art can also be used as anti-fungal agents or anti-oxidants.

An alternative method of producing the probiotic preparation which does not require an active growing step simply involves inoculated the growth substrate (e.g. the extract of germinated barley prepared as describe herein) with starter culture(s) of probiotic bacteria. Starter cultures for the method include, for example, freeze-dried bacteria or liquid cultures. The growth substrate may be inoculated with more than one bacterial species. Preferably, the concentration of the viable cells in the starter culture is in excess of 10⁶ viable cells per millilitre.

The invention will be further understood with reference to the following non-limiting experimental examples:

### Example 1: Production of a growth substrate, using a barley-based medium.

### (A) Germination (malting)

### Day 1

Barley (seed or malting sample grains) was steeped in water for 2 to 24 hours, depending on the batch of grain. 0.1% (w/v) sodium hypochlorite (bleach) can be included in the water to inhibit growth of contaminants during the germination phase. After 4 hours, the water was drained from the grains and the grains left to stand at room temperature from 10 to 30°C for approximately 1 day.

### Day 2

The grains were steeped in clean water for another 4-hour period. Hydrogen peroxide (0.1% w/v) may be added to the water for this and subsequent soaks. Hydrogen peroxide provides oxygen for the germinating grains, and act as a disinfectant. Alternatively, sodium hypochlorite may be used. After 4 hours, water was drained from the grains and the grains left to stand for approximately 1 day. Occasional (e.g. every 4 hours) agitation of the grains may improve germination by increasing gaseous exchange, providing oxygen and removing carbon dioxide.

### Day 3 onwards

The cycle of steeping, draining and standing was continued until the emerging rootlets on the germinating grains were 2 to 4 mm long. This state of growth indicated that the grains had produced enzymes for the mobilisation of stored nutrients. These enzymes are central to the subsequent production of the growth medium, during 'mashing-in.' More extensive modification of the grains could be allowed, leaving the germination phase until the rootlets are several millimetres long. However, too much growth will merely convert nutrients into plant roots and shoots, which cannot be used in the fermentation.

### (B) Rolling

When the grains had germinated sufficiently, they were then milled with a roller mill. The mill was adjusted such that the grains were cracked open but not shattered or completely flattened. Cracking of the grains allows access of water and extraction of nutrients during mashing in, whilst avoiding shattering of the grains assists in filtration steps.

### (C) Preparation of a growth substrate.

The germinated, milled, grains were mixed with sufficient water to cover them, at 45°C, and the mixture held at 45°C for 1 hour.

After 1 hour at 45°C, the temperature was increased to 78° C over a period of 30 minutes.

The mixture was allowed to stand at 78°C for 1 hour. After the 78°C stand, the spent grains were separated out by filtration. A relatively coarse filter (e.g. 1mm gap wedge-wire basket) was used yielding a solution containing significant amounts of suspended solids. The spent grains were discarded and the liquid then heat-treated to pasteurise or sterilise it. In this particular experiment the liquid was boiled for 45 minutes. A buffer (0.5% (w/v) tri-sodium citrate) was then added and the mixture boiled for a further 15 minutes to yield the final growth substrate.

### Example 2: Analysis of the growth substrate

### (a) Carbohydrate Analyses:

Total carbohydrate content was determined using the phenol-sulphuric acid assay, with glucose as reference standard. (Dubois, M., Gilles, K. A., Hamilton, J. K., Rebers, P. A. and Smith, F. (1956) Analytical Chemistry, vol. 28., p. 350).

Reducing sugars were determined using the Nelson-Somogyi method, again with glucose as the reference standard. (Somogyi, M. (1952) Journal of Biological Chemistry., vol. 195., p. 19). Both complex and simple sugars have reducing ends, but simple sugars have a greater number of reducing ends per unit mass. Thus, whilst the Nelson-Somogyi method does detect complex sugars, the signal is so low so as to be negligible. The Nelson-Somogyi method therefore gives a good approximation of the level of simple sugars in a sample.

### Results:

Total sugars in substrate are in the range 20 to 40 mg/ml (milligrams per millilitre)
Reducing sugars are in the range 5 to 20 mg/ml.

### (b) Protein and Peptide Analyses:

Total protein was determined using two assays, with bovine serum albumin as reference standard:
(i) The Biuret Reagent (Itzhaki, R. F & Gill, D. M. (1964) Analytical Biochemistry, Vol. 9., p. 401-410.
(ii) The Lowry Method, as modified by Ohnishi and Barr. (Ohnishi, S. T. & Barr, J. K. (1978) Journal of Biological Chemistry, Vol. 193, p. 265).

Peptides of molecular weight above 5000 daltons were determined using the Bradford Reagent (Bradford, M. M. (1976) Analytical Biochemistry, Vol. 72, p. 248-254).

### Results:

Total protein and peptides are in the range 1 to 2 milligrams per millilitre.

High molecular weight peptides (greater than 5000 daltons) are in the range 100 to 300 micrograms per millilitre.

### Example 3: Production of a metabolically active bacterial culture

### (A) Fermentation

The growth substrate prepared according to example 1 was cooled to 37°C and the bacterial cultures added. Examples of a suitable inoculum are freeze-dried bacteria or liquid starter cultures (typically 1% (v/v) of an overnight culture in nutrient broth).

In this example the following bacteria were grown in two vessels:
*(i) Enterococcus faecium, Lactobacillus plantarum;*
*(ii)* A strain of *Lactobacillus casei* which has now been re-classified as *Lactobacillus rhamnosus, Lactobacillus acidophilus*

Fermentation was carried out for 16-20 hours, until the pH reached 4.5 +/- 0.3 units. The fermentation mixture was then cooled to 4°C and subjected standard techniques to assess quality (to determine microbiological purity and enumeration of bacteria).

At this point, sterile potassium sorbate (0.005% w/v final concentration) may be added to the fermented broth. This acts to inhibit the growth of any fungi or yeast that may arise due to contamination during handling by the end-user.

Vitamin C may also be added (0.01% w/v final concentration) as an anti-oxidant.

Following quality assurance tests, different batches can be blended to give products with complex mixtures of bacterial species, if required.

In the example presented here, blending of the two batches of bacteria yielded a final product containing the bacteria *Enterococcus faecium, Lactobacillus plantarum, Lactobacillus acidophilus* and the strain of *Lactobacillus casei* which has now been re-classified as *Lactobacillus rhamnosus.*

Batches of this final product typically contain 2.3g carbohydrate per 100ml, of which 0.7g is sugars and 1.6g is starch. The bacterial count in the final product is typically less than 1.0 x 10⁵ for *L. acidophilus* and typically at least 1.0 x 10⁷ for each of, *L. plantarum, E. faecium* and the strain of *Lactobacillus casei* which has now been re-classified as *Lactobacillus rhamnosus.*

Batches of the final product were tested for the presence of gluten using the Gliadin ELISA test and were determined to contain less than 15ppm gluten, and more typically less than 10ppm gluten, satisfying the Codex Alimentarius definition of gluten-free.

The Symprove^{™} product described herein is commercially available from Multigerm UK Enterprises Ltd.

### Example 4: Effective treatment of enterocolitis, as measured by faecal calprotectin

A randomized, double-blinded, placebo controlled trial was undertaken to assess if the probiotic Symprove^{™} (a multistrain water based probiotic containing *L. rhamnosus, E. faecium, L. acidophilus,* and *L. plantarum,* prepared according to the method described herein and commercially available from Multigerm UK Enterprises Ltd) taken for 1 month reduced intestinal inflammation (as assessed by a faecal calprotectin test) in patients with inflammatory bowel disease (ulcerative colitis and Crohn's disease) in clinical remission with only mild symptoms.

Eighty patients with UC and 63 with CD were recruited. Patients included were aged 18-64, had a diagnosis of mild to moderate inflammatory bowel disease (Crohn's disease' or ulcerative colitis) that was confirmed both endoscopically (ileo-colonoscopy) and histologically (ileal or colonic biopsy). Duration of IBD must have been at least 6 months with 3 months in 'stable clinical remission' prior to inclusion. "Stable clinical remission" was defined as "patients whose symptoms are relatively mild and show no progressive deterioration and in whom changes to current treatment is not indicated". Patients who had moderate to severe disease; were currently receiving biological treatments; had significant co-morbidities including psychiatric conditions, previous complicated bowel resection; documented allergy or intolerance of probioitics; were pregnant or actively seeking pregnancy; had a history of drug or alcohol dependence were excluded. Patients receiving immunosuppressive therapy (azathioprine or 6 mercaptopurine) were not excluded if they had been stable on treatment without major side effects or adverse events for a minimum of 12 weeks.

Faecal calprotectin was assessed before and after being randomized (2 stage computer randomisation utilising the Mersenne Algorithm) to receive the probiotic or a matching placebo (1 ml/kg body weight daily for one month).

Patients took 1mg/kg of probiotic or placebo (having no probiotic activity) orally each morning on an empty stomach, with no food or drink for 20 minutes post-dose.

Handling of stool samples (each 200mg or more) and calprotectin measurement was performed by ELISA using the Calprest commercially available kit. This assay has an intra-assay variation of 2% and inter-assay variation of <15%, with normal (i.e. healthy) values being <50mg/kg.

**Results:** In patients with UC and Crohn's disease that were treated with the probiotic, the mean faecal calprotectin concentration fell significantly compared to placebo (Table 1):

**Table 1**

| | Ulcerative colitis | | |
|---|---|---|---|
| | Before treatment (mg/kg) | After treatment (mg/kg) | Mean reduction |
| Probiotic | 725 | 411 | 314 |
| Placebo | 418 | 627 | -209 |

| | Crohn's Disease | | |
|---|---|---|---|
| | Before treatment (mg/kg) | After treatment (mg/kg) | Mean reduction |
| Probiotic | 743 | 531 | 189 |
| Placebo | 263 | 573 | -270 |

| | | | |
|---|---|---|---|
| *"Mean reduction" is the average reduction in faecal calprotectin concentration per patient as a result of the indicated intervention. A negative value indicates a mean increase in concentration as a result of the intervention.* | | | |

In addition, when the reduction in faecal calprotectin concentration was measured as a percentage of the faecal calprotectin concentration of each patient prior to treatment, ulcerative colitis patients that received the probiotic exhibited a mean percentage reduction in calprotectin level of 21% compared to a mean percentage reduction of -101% (i.e. an increase of approximately 2x the calprotectin level before treatment) in UC patients that received placebo.

These data show that the probiotic preparation comprising viable, metabolically active probiotic bacteria in a non-dairy liquid substrate as described herein provides effective treatment of entercolitic disorders such as IBD (UC and Crohn's disease) by reducing the levels of inflammation in the gut, as indicated by an absolute and relative reduction in faecal calprotectin concentration. As a result of the placebo control group, the observed therapeutic effects can be reliably attributed to the probiotic preparation. Indeed, the observed changes in calprotectin concentration in the placebo groups indicate that an increase in calprotectin concentration would be expected if the probiotic was having no effect. However, treatment with the probiotic preparation results in a reduction in faecal calprotectin level, clearly indicating a therapeutic effect on the underlying enterocolitic disorder. It is expected that the probiotic preparation would have the same or similar therapeutic effects on other enterocolitic disorders characterised by increased levels of faecal calprotectin.

## Claims

1. A probiotic preparation comprising a mixture of viable, metabolically active *Enterococcus faecium, Lactobacillus plantarum, Lactobacillus acidophilus,* and either *Lactobacillus casei* or *Lactobacillus rhamnosus* or both *Lactobacillus casei* and *Lactobacillus rhamnosus* in a non-dairy liquid substrate, for use in the treatment of enterocolitis in a patient having a faecal calprotectin level of greater than 50mg/kg.

2. The probiotic preparation for use according to claim 1 for use in the treatment of inflammatory bowel disease (IBD), optionally for use in the treatment of Crohn's disease or ulcerative colitis.

3. The probiotic preparation for use according to any preceding claim, wherein the patient is in clinical remission, optionally wherein the patient has been in clinical remission for at least 3 months.

4. The probiotic preparation for use according to any preceding claim for use in the treatment of a patient having a faecal calprotectin level of greater than 250mg/kg, optionally greater than 700mg/kg.

5. The probiotic preparation for use according to any preceding claim, wherein the probiotic preparation comprises a mixture of complex carbohydrates and simple sugars, wherein the ratio of total carbohydrate content to reducing sugar content of the probiotic preparation is in the range of from 8:1 to 2:1.

6. The probiotic preparation for use according to any preceding claim, wherein the probiotic preparation has a total carbohydrate content in the range of from 20 mg/ml to 40 mg/ml and a reducing sugar content in the range of from 5 mg/ml to 20 mg/ml.

7. The probiotic preparation for use according to any one of the preceding claims which additionally comprises proteins and peptides, wherein the total amount of protein and peptides is in the range of from 1 mg/ml to 2 mg/ml and wherein the total amount of high molecular weight peptides is in the range of from 100 µg/ml to 300 µg/ml.

8. The probiotic preparation for use according to any of the preceding claims wherein the probiotic preparation is prepared by growing the probiotic bacterial strain(s) in an extract of germinated barley.

9. A probiotic preparation for use in the treatment of enterocolitis in a patient having a faecal calprotectin level of greater than 50mg/kg, the probiotic preparation being obtainable by a method comprising:
subjecting malted cereal, optionally barley, to a mashing step to obtain a mixture of complex carbohydrates, simple sugars, proteins and peptides;
separating the mixture of complex carbohydrates and simple sugars, proteins and peptides from the spent malted cereal to obtain a growth substrate;
inoculating the growth substrate with a mixture of *Enterococcus faecium, Lactobacillus plantarum, Lactobacillus acidophilus* and either *Lactobacillus casei* or *Lactobacillus rhamnosus* or both *Lactobacillus casei* and *Lactobacillus rhamnosus* to form a culture and growing said bacteria until they reach a concentration of between 1 x 10⁶ and 1 x 10⁹ colony forming units per millilitre;
cooling the culture to 4°C to obtain the probiotic preparation.

10. The probiotic preparation for use according to claim 9 for use in the treatment of inflammatory bowel disease (IBD), optionally for use in the treatment of Crohn's disease or ulcerative colitis.

11. The probiotic preparation for use according to any one of claims 9-10, wherein the patient is in clinical remission, optionally wherein the patient has been in clinical remission for at least 3 months.

12. The probiotic preparation for use according to any one of claims 9-11 for use in the treatment of a patient having a faecal calprotectin level of greater than 250mg/kg, optionally greater than 700mg/kg.

13. The probiotic preparation for use according to any one of the preceding claims, wherein the total count of at least one of, and more preferably each of *Enterococcus faecium, Lactobacillus plantarum,* and either *Lactobacillus casei* or *Lactobacillus rhamnosus* or a combination of *Lactobacillus casei* and *Lactobacillus rhamnosus* in the preparation is at least 1.0 x 10⁶ viable cells per millilitre, preferably at least 1.0 x 10⁷ viable cells per millilitre.

14. The probiotic preparation for use according to any one of the preceding claims, wherein the total count of metabolically active bacteria in the preparation is in the range of from 1.0 x 10⁶ to 1.0 x 10⁹ viable cells per millilitre.

## Patentansprüche

1. Probiotisches Präparat, umfassend eine Mischung von lebensfähigen, metabolisch aktiven *Enterococcus faecium, Lactobacillus plantarum, Lactobacillus acidophilus,* und entweder *Lactobacillus casei* oder *Lactobacillus rhamnosus* oder sowohl *Lactobacillus casei* als auch *Lactobacillus rhamnosus* in einem nicht-milchhaltigen flüssigen Substrat, zur Verwendung bei der Behandlung von Enterokolitis in einem Individuum mit einem fäkalen Calprotectinwert von mehr als 50 mg/kg.

2. Probiotisches Präparat zur Verwendung nach Anspruch 1 zur Verwendung bei der Behandlung von chronisch-entzündlicher Darmerkrankung (IBD), gegebenenfalls zur Verwendung bei der Behandlung von Morbus Crohn oder Colitis ulcerosa.

3. Probiotisches Präparat zur Verwendung nach einem der vorhergehenden Ansprüche, wobei das Individuum sich in klinischer Remission befindet, gegebenenfalls wobei das Individuum sich für mindestens 3 Monate in klinischer Remission befunden hat.

4. Probiotisches Präparat zur Verwendung nach einem der vorhergehenden Ansprüche zur Verwendung bei der Behandlung eines Individuums mit einem fäkalen Calprotectinwert von mehr als 250 mg/kg, gegebenenfalls mehr als 700 mg/kg.

5. Probiotisches Präparat zur Verwendung nach einem der vorhergehenden Ansprüche, wobei das probiotische Präparat eine Mischung komplexer Kohlenhydrate und einfacher Zucker umfasst, wobei das Verhältnis des Gesamtkohlenhydratgehalts zum reduzierenden Zuckergehalt des probiotischen Präparats im Bereich von 8:1 zu 2:1 liegt.

6. Probiotisches Präparat zur Verwendung nach einem der vorhergehenden Ansprüche, wobei das probiotische Präparat einen Gesamtkohlenhydratgehalt im Bereich von 20 mg/ml bis 40 mg/ml und einen reduzierenden Zuckergehalt im Bereich von 5 mg/ml bis 20 mg/ml aufweist.

7. Probiotisches Präparat zur Verwendung nach einem der vorhergehenden Ansprüche, welches zusätzlich Proteine und Peptide umfasst, wobei der Gesamtgehalt an Proteinen und Peptiden im Bereich von 1 mg/ml bis 2 mg/ml liegt und der Gesamtgehalt von Peptiden mit hohem Molekulargewicht im Bereich von 100 µg/ml bis 300 µg/ml liegt.

8. Probiotisches Präparat zur Verwendung nach einem der vorhergehenden Ansprüche, wobei das probiotische Präparat durch Wachsen des/der probiotischen Bakterienstamms/Bakterienstämme in einem Extrakt von gekeimter Gerste hergestellt wird.

9. Probiotisches Präparat zur Verwendung bei der Behandlung von Enterokolitis in einem Individuum mit einem fäkalen Calprotectinwert von mehr als 50 mg/kg, wobei das probiotische Präparat erhältlich ist durch ein Verfahren umfassend:
Aussetzen von Getreidemalz, gegebenenfalls Gerste, einem Maischeschritt, um eine Mischung von komplexen Kohlenhydraten, einfachen Zuckern, Proteinen und Peptiden zu erhalten;
Trennen der Mischung von komplexen Kohlenhydraten und einfachen Zuckern, Proteinen und Peptiden von dem verbrauchten Getreidemalz, um ein Wachstumssubstrat zu erhalten;
Inokulieren des Wachstumssubstrats mit einer Mischung aus *Enterococcus faecium, Lactobacillus plantarum, Lactobacillus acidophilus* und entweder *Lactobacillus casei* oder *Lactobacillus rhamnosus* oder sowohl *Lactobacillus casei* als auch *Lactobacillus rhamnosus* um eine Kultur zu bilden und Wachsen der Bakterien bis sie eine Konzentration von zwischen 1 x 10⁶ und 1 x 10⁹ koloniebildende Einheiten pro Milliliter erreichen;
Abkühlen der Kultur auf 4 °C um das probiotische Präparat zu erhalten.

10. Probiotisches Präparat zur Verwendung nach Anspruch 9 zur Verwendung bei der Behandlung von chronisch-entzündlicher Darmerkrankung (IBD), gegebenenfalls zur Verwendung bei der Behandlung von Morbus Crohn oder Colitis ulcerosa.

11. Probiotisches Präparat zur Verwendung nach einem der Ansprüche 9-10, wobei das Individuum sich in klinischer Remission befindet, gegebenenfalls wobei das Individuum sich für mindestens 3 Monate in klinischer Remission befunden hat.

12. Probiotisches Präparat zur Verwendung nach einem der Ansprüche 9-11, zur Verwendung bei der Behandlung eines Individuums mit einem fäkalen Calprotectinwert von mehr als 250 mg/kg, gegebenenfalls mehr als 700 mg/kg.

13. Probiotisches Präparat zur Verwendung nach einem der vorhergehenden Ansprüche, wobei die Gesamtzahl von mindestens einem aus und stärker bevorzugt allen aus *Enterococcus faecium, Lactobacillus plantarum,* und entweder *Lactobacillus casei* oder *Lactobacillus rhamnosus* oder eine Kombination von *Lactobacillus casei* und *Lactobacillus rhamnosus* in dem Präparat mindestens 1.0 x 10⁶ lebensfähige Zellen pro Milliliter, bevorzugt mindestens 1.0 x 10⁷ lebensfähige Zellen pro Milliliter beträgt.

14. Probiotisches Präparat zur Verwendung nach einem der vorhergehenden Ansprüche, wobei die Gesamtzahl metabolisch aktiver Bakterien in dem Präparat im Bereich von 1.0 x 10⁶ bis 1.0 x 10⁹ lebensfähigen Zellen pro Milliliter liegt.

## Revendications

1. Préparation probiotique comprenant un mélange *d'Enterococcus faecium,* de *Lactobacillus plantarum,* de *Lactobacillus acidophilus* et de *Lactobacillus casei* ou de *Lactobacillus rhamnosus* ou à la fois de *Lactobacillus casei* et de *Lactobacillus rhamnosus* viables et métaboliquement actives dans un substrat liquide non laitier, pour une utilisation dans le traitement d'entérocolite chez un patient ayant un taux de calprotectine fécale supérieur à 50 mg/kg.

2. Préparation probiotique à utiliser selon la revendication 1 pour une utilisation dans le traitement d'une maladie inflammatoire de l'intestin (IBD), facultativement pour une utilisation dans le traitement de la maladie de Crohn ou d'une colite ulcéreuse.

3. Préparation probiotique à utiliser selon l'une quelconque des revendications précédentes, dans laquelle le patient est en rémission clinique, facultativement dans laquelle le patient est en rémission clinique depuis au moins 3 mois.

4. Préparation probiotique à utiliser selon l'une quelconque des revendications précédentes, pour une utilisation dans le traitement d'un patient ayant un taux de calprotectine fécale supérieur à 250 mg/kg, facultativement supérieur à 700 mg/kg.

5. Préparation probiotique à utiliser selon l'une quelconque des revendications précédentes, dans laquelle la préparation probiotique comprend un mélange de glucides complexes et de sucres simples, dans laquelle le rapport entre la teneur totale en glucides et la teneur en sucres réducteurs de la préparation probiotique est compris entre 8:1 et 2:1.

6. Préparation probiotique à utiliser selon l'une quelconque des revendications précédentes, dans laquelle la préparation probiotique a une teneur totale en glucides comprise entre 20 mg/ml et 40 mg/ml et une teneur en sucres réducteurs comprise entre 5 mg/ml et 20 mg/ml.

7. Préparation probiotique à utiliser selon l'une quelconque des revendications précédentes, qui comprend de plus des protéines et des peptides, dans laquelle la quantité totale de protéines et de peptides est comprise entre 1 mg/ml et 2 mg/ml et dans laquelle la quantité totale de peptides de poids moléculaire élevé est comprise entre 100 µg/ml et 300 µg/ml.

8. Préparation probiotique à utiliser selon l'une quelconque des revendications précédentes, dans laquelle la préparation probiotique est préparée en faisant croître la ou les souches bactériennes probiotiques dans un extrait d'orge germé.

9. Préparation probiotique à utiliser dans le traitement d'entérocolite chez un patient ayant un taux de calprotectine fécale supérieur à 50 mg/kg, la préparation probiotique pouvant être obtenue par un procédé comprenant les étapes consistant à :
soumettre des céréales maltées, facultativement de l'orge, à une étape de brassage pour obtenir un mélange de glucides complexes, de sucres simples, de protéines et de peptides ;
séparer le mélange de glucides complexes et de sucres simples, de protéines et de peptides vis-à-vis de la céréale maltée usée pour obtenir un substrat de mise en croissance ;
inoculer le substrat de mise en croissance avec un mélange d'*Enterococcus faecium,* de *Lactobacillus plantarum,* de *Lactobacillus acidophilus* et de *Lactobacillus casei* ou de *Lactobacillus rhamnosus* ou à la fois de *Lactobacillus casei* et de *Lactobacillus rhamnosus* pour former une culture et faire croître lesdites bactéries jusqu'à ce qu'elles atteignent une concentration comprise entre 1 x 10⁶ et 1 x 10⁹ unités formant colonies par millilitre ;
refroidir la culture à 4 °C pour obtenir la préparation probiotique.

10. Préparation probiotique à utiliser selon la revendication 9 pour une utilisation dans le traitement d'une maladie inflammatoire de l'intestin (IBD), facultativement pour une utilisation dans le traitement de la maladie de Crohn ou d'une colite ulcéreuse.

11. Préparation probiotique à utiliser selon l'une quelconque des revendications 9 à 10, dans laquelle le patient est en rémission clinique, facultativement dans laquelle le patient est en rémission clinique depuis au moins 3 mois.

12. Préparation probiotique à utiliser selon l'une quelconque des revendications 9 à 11 pour une utilisation dans le traitement d'un patient ayant un taux de calprotectine fécale supérieur à 250 mg/kg, facultativement supérieur à 700 mg/kg.

13. Préparation probiotique à utiliser selon l'une quelconque des revendications précédentes, dans laquelle le nombre total d'au moins l'une parmi, et de manière plus préférée de chacune parmi, *Enterococcus faecium, Lactobacillus plantarum* et *Lactobacillus casei* ou *Lactobacillus rhamnosus* ou une combinaison de *Lactobacillus casei* et de *Lactobacillus rhamnosus* dans la préparation est d'au moins 1,0 x 10⁶ cellules viables par millilitre, de préférence d'au moins 1,0 x 10⁷ cellules viables par millilitre.

14. Préparation probiotique à utiliser selon l'une quelconque des revendications précédentes, dans laquelle le nombre total de bactéries métaboliquement actives dans la préparation est compris entre 1,0 x 10⁶ et 1,0 x 10⁹ cellules viables par millilitre.
